(19) Europäisches Patentamt
European Patent Office
Office européen des brevets

(11) Veröffentlichungsnummer: **0 087 032**
**B1**

(12) # EUROPÄISCHE PATENTSCHRIFT

(45) Veröffentlichungstag der Patentschrift: **08.05.85**

(21) Anmeldenummer: **83101088.9**

(22) Anmeldetag: **05.02.83**

(51) Int. Cl.[4]: **C 07 C 69/753**, C 09 K 19/00, C 07 C 67/08, C 07 C 69/24, C 07 D 309/06

(54) **Bicyclohexylderivatemit flüssig-kristallinen Eigenschaften.**

(30) Priorität: **20.02.82 DE 3206269**

(43) Veröffentlichungstag der Anmeldung: **31.08.83 Patentblatt 83/35**

(45) Bekanntmachung des Hinweises auf die Patenterteilung: **08.05.85 Patentblatt 85/19**

(84) Benannte Vertragsstaaten: **AT CH DE FR GB IT LI NL**

(56) Entgegenhaltungen: **EP - A - 0 003 215** **DE - A - 2 747 113**

(73) Patentinhaber: **Merck Patent Gesellschaft mit beschränkter Haftung, Frankfurter Strasse 250, D-6100 Darmstadt (DE)**

(72) Erfinder: **Eidenschink, Rudolf, Dr., Erlenweg 17, D-6110 Dieburg (DE)**
Erfinder: **Krause, Joachim, Dr., Samuel-Morse-Strasse 14, D-6110 Dieburg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die Erfindung betrifft neue Bicyclohexylderivate der Formel I

$$R^1-Cy-Cy-X-Y \quad (I)$$

worin

X $-CO-O-$ oder $-O-CO-$ und
Y Alkyl oder Perfluoralkyl mit jeweils 1—8 C-Atomen, $-Cy-Cy-R^2$, $-Cy-Ph-R^2$, $-Ph-Cy-R^2$, $-Ph-Ph-R^2$ oder $-Ph-Dio-R^2$,
Cy 1,4-Cyclohexylen,
Ph 1,4-Phenylen,
Dio 1,3-Dioxan-2,5-diyl und
$R^1$ und $R^2$ jeweils Alkyl oder Alkoxy mit jeweils 1—8 C-Atomen

bedeuten.

Diese Substanzen können wie ähnliche, z. B. aus der DE-OS 2 800 553 bekannte Verbindungen als Komponenten flüssig-kristalliner Dielektrika verwendet werden, insbesondere für Displays, die auf dem Prinzip der verdrillten Zelle beruhen.

Der Erfindung lag die Aufgabe zugrunde, neue flüssigkristalline Verbindungen aufzufinden, die als Komponenten flüssig-kristalliner Dielektrika geeignet sind, insbesondere für nematische Phasen mit niedriger optischer Anisotropie. Diese Aufgabe wurde durch die Bereitstellung der Verbindungen der Formel I gelöst.

Es wurde gefunden, daß die Verbindungen der Formel I vorzüglich als Komponenten flüssigkristalliner Dielektrika geeignet sind. Insbesondere sind mit ihrer Hilfe flüssigkristalline Phasen mit relativ niedriger optischer Anisotropie herstellbar, wie sie für verdrillte Zellen im Sub-Mauguin-Bereich zur Erzielung eines wenig winkelabhängigen Kontrastes benötigt werden (vgl. DE-OS 3 022 818).

Die Verbindungen der Formel I sind in reinem Zustand farblos und bilden flüssigkristalline Mesophasen in einem für die elektrooptische Verwendung günstig gelegenen Temperaturbereich.

Diejenigen Verbindungen der Formel I, die optisch aktiv sind, eignen sich als chirale Dotierstoffe zur Herstellung cholesterischer Phasen wie sie für Farbstoff-Zellen nach White-Taylor verwendet werden können. Gegenüber den bisher üblichen Biphenylderivaten zeigen sie die Vorteile einer höheren »Twisting Power« und einer geringeren optischen Anisotropie; in niedrigen Konzentrationen vermögen diese chiralen Verbindungen in der verdrillten Zelle Störungen des optischen Erscheinungsbildes durch den »umgekehrten Twist« zu verhindern (vgl. Mol. Cryst. Liq. Cryst., Band 34 (Letters), Seiten 211—217 (1977)).

Gegenstand der Erfindung sind somit die Verbindungen der Formel I sowie ein Verfahren zu ihrer Herstellung, dadurch gekennzeichnet, daß man eine Carbonsäure der Formel II

$$Q-COOH \quad (II)$$

worin

Q (a) $R^1-Cy-Cy-$ oder
(b) Y bedeutet und
$R^1$, Cy und Y die angegebene Bedeutung haben

oder eines ihrer reaktionsfähigen Derivate mit einer Hydroxyverbindung der Formel III

$$Z-OH \quad (III)$$

worin

$Z_1$ (a) Y oder
(b) $R^1-Cy-Cy-$ bedeutet und
$R^1$, Cy und Y die angegebene Bedeutung haben

oder einem ihrer reaktionsfähigen Derivate umsetzt
oder daß man zur Herstellung von Verbindungen der Formel I ($Y = -Ph-Dio-R^2$) einen Aldehyd der Formel IV

$$R^1-Cy-Cy-X-Ph-CHO \quad (IV)$$

worin $R^1$, X, Ph und Cy die angegebene Bedeutung haben

mit einem Diol der Formel V

$(HOCH_2)_2-CH-R^2$ (V)

worin $R^2$ die angegebene Bedeutung hat umsetzt. Weiterhin sind Gegenstand der Erfindung die Verwendung der Verbindungen der Formel I als Komponenten flüssigkristalliner Dielektrika, flüssigkristalline Dielektrika mit einem Gehalt an mindestens einer Verbindung der Formel I sowie elektrooptische Anzeigeelemente, die derartige Dielektrika enthalten.

Vor- und nachstehend haben X, Y, Cy, Ph, Dio, $R^1$, $R^2$, Q und Z die angegebene Bedeutung.

Die Verbindungen der Formel I umfassen Ester der Formeln Ia und Ib

$R^1-Cy-Cy-CO-O-Y$ (Ia)

$R^1-Cy-Cy-O-CO-Y$ (Ib)

Im einzelnen umschließt die Formel I Ester der folgenden Formeln Ic bis Ip

$R^1-Cy-Cy-CO-O-Alkyl$ (Ic)

$R^1-Cy-Cy-CO-O-Perfluoralkyl$ (Id)

$R^1-Cy-Cy-CO-O-Cy-Cy-R^2$ (Ie)

$R^1-Cy-Cy-CO-O-Cy-Ph-R^2$ (If)

$R^1-Cy-Cy-CO-O-Ph-Cy-R^2$ (Ig)

$R^1-Cy-Cy-CO-O-Ph-Ph-R^2$ (Ih)

$R^1-Cy-Cy-CO-O-Ph-Dio-R^2$ (Ii)

$R^1-Cy-Cy-O-CO-Alkyl$ (Ij)

$R^1-Cy-Cy-O-CO-Perfluoralkyl$ (Ik)

$R^1-Cy-Cy-O-CO-Cy-Cy-R^2$ (Il)

$R^1-Cy-Cy-O-CO-Cy-Ph-R^2$ (Im)

$R^1-Cy-Cy-O-CO-Ph-Cy-R^2$ (In)

$R^1-Cy-Cy-O-CO-Ph-Ph-R^2$ (Io)

$R^1-Cy-Cy-O-CO-Ph-Dio-R^2$ (Ip)

worin die Alkyl-, Perfluoralkyl- und Alkoxygruppen jeweils 1—8, vorzugsweise 1—5, insbesondere 3, 4 oder 5 C-Atome enthalten.

Die Verbindungen der Formel Ic, ferner diejenigen der Formeln Id bis Ig, Ii, Ik bis In sowie Ip sind bevorzugt.

Die Reste $R^1$ und $R^2$ sind vorzugsweise Alkyl.

In den Verbindungen der Formel I sowie Ia bis Ip sind diejenigen Stereoisomeren bevorzugt, worin die beiden Substituenten an den Cyclohexylen- und den Dioxandiylresten jeweils in trans-Stellung zueinander stehen.

In den Verbindungen der Formel I sowie Ia bis Ip sind die Alkyl-, Alkoxy- und Perfluoralkylgruppen vorzugsweise geradkettig. Alkyl bedeutet also vorzugsweise Methyl, Ethyl, n-Propyl, n-Butyl, n-Pentyl, n-Hexyl, n-Heptyl oder n-Octyl; Perfluoralkyl bedeutet vorzugsweise Trifluormethyl, Pentafluorethyl, Heptafluorpropyl, Nonafluorbutyl, Undekafluorpentyl, Tridekafluorhexyl, Pentadekafluorheptyl oder Heptadekafluoroctyl; Alkoxy bedeutet vorzugsweise Methoxy, Ethoxy, n-Propyloxy, n-Butyloxy, n-Pentyloxy, n-Hexyloxy, n-Heptyloxy oder n-Octyloxy.

Verbindungen der Formel I sowie Ia bis Ip mit verzweigten Alkyl-, Perfluoralkyl- oder Alkoxygruppen können gelegentlich wegen einer besseren Löslichkeit in dem üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Alkylreste sind Isopropyl, 2-Butyl (= 1-Methylpropyl), Isobutyl (= 2-Methyl-propyl),

2-Methyl-butyl, Isopentyl (= 3-Methylbutyl), 2-Methylpentyl, 3-Methylpentyl, 2-Ethylhexyl, 2-Heptyl (= 1-Methylhexyl), 2-Octyl (= 1-Methylheptyl); bevorzugte verzweigte Alkoxyreste sind Isopropyloxy, 2-Methyl-propyloxy, 2-Methylbutyloxy, 3-Methyl-butyloxy, 2-Methyl-pentyloxy, 3-Methyl-pentyloxy, 2-Ethyl-hexyloxy, 1-Methyl-hexyloxy, 1-Methyl-heptyloxy.

Die Verbindungen der Formel I werden im übrigen nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Als reaktionsfähige Derivate der Carbonsäuren der Formel II eignen sich insbesondere die Säurehalogenide, vor allem die Chloride und Bromide, ferner die Anhydride, z. B. auch gemischte Anhydride der Formel

$$Q-CO-O-COCH_3,$$

Azide, Ester, insbesondere Alkylester mit 1—4 C-Atomen in der Alkylgruppe.

Als reaktionsfähige Derivate der Alkohole bzw. Phenole der Formel III kommen insbesondere die entsprechenden Metall-alkoholate bzw. -phenolate der Formel Z—OM in Betracht, in der M eine Äquivalent eines Metalls, vorzugsweise eines Alkalimetalls wie Na oder K, bedeutet.

Die Veresterung wird vorteilhaft in Gegenwart eines inerten Lösungsmittels durchgeführt. Gut geeignet sind insbesondere Ether wie Diethylether, Di-n-butylether, Tetrahydrofuran, Dioxan oder Anisol, Ketone wie Aceton, Butanon oder Cyclohexanon, Amide wie Dimethylformamid oder Phosphorsäure-hexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, zum Beispiel Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat durchgeführt werden.

Die Reaktionstemperatur liegt gewöhnlich zwischen —50° und +250°, vorzugsweise zwischen —20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure der Formel II mit einem Alkohol oder Phenol der Formel III in der Regel in Gegenwart einer starken Säure, beispielsweise einer Mineralsäure wie Salzsäure oder Schwefelsäure, umgesetzt.

Eine bevorzugte Reaktionszeit ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind.

Eine weitere bevorzugte Ausführungsform des Verfahrens nach der Erfindung besteht darin, daß man die zu veresternde Hydroxyverbindung der Formel III zunächst in das Natrium- oder Kaliumalkoholat bzw. -phenolat überführt, z. B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder Dimethylformamid versetzt, zweckmäßig bei Temperaturen zwischen etwa —25° und +20°.

Zur Herstellung der Phenyldioxanderivate der Formel I ($Y = -Ph-Dio-R^2$) kann man auch einen Aldehyd der Formel IV mit einem Diol der Formel V acetalisieren, zweckmäßig in Gegenwart eines inerten Lösungsmittels wie Benzol oder Toluol und/oder eines Katalysators, z. B. einer starken Säure wie Schwefelsäure, Benzol- oder p-Toluolsulfonsäure, bei Temperaturen zwischen etwa 20 und etwa 150, vorzugsweise zwischen 80 und 120°.

Die Ausgangsstoffe der Formeln II bis V sowie ihre reaktionsfähigen Derivate sind zum großen Teil bekannt, zum Teil können sie ohne Schwierigkeiten nach Standardverfahren der organischen Chemie aus literaturbekannten Verbindungen hergestellt werden.

Die erfindungsgemäßen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder

Cyclohexylbenzoate, Cyclohexancarbonsäurephenyl- oder -cyclohexylester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexyl-pyrimidine, Phenyl- oder Cyclohexyldioxane, gegebenenfalls halogenierte Stilbene, Benzylphenylether, Tolane und substituierten Zimtsäuren. Die wichtigsten als Bestandteile derartiger flüssigkristalliner Dielektrika in Frage kommenden Verbindungen lassen sich durch die Formel VI charakterisieren,

$$R^3-A-G-E-R^4 \qquad (VI)$$

worin A und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe.

| | | |
|---|---|---|
| G | $-CH=CH-$ | $-N(O)=N-$ |
| | $-CH=CL-$ | $-CH=N(O)-$ |
| | $-C\equiv C-$ | $-CH_2-CH_2-$ |
| | $-CO-O-$ | $-CH_2-O-$ |
| | $-CO-S-$ | $-CH_2-S-$ |
| | $-CH=N-$ | $-COO-Ph-COO-$ |

oder eine C—C-Einfachbindung,

L Halogen, vorzugsweise Chlor, oder CN, und $R^3$ und $R^4$ Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten. Bei den meisten dieser Verbindungen sind $R^3$ und $R^4$ voneinander verschieden, wobei einer dieser Reste meist eine Alkyl- oder Alkoxygruppe ist. Viele solcher Substanzen oder auch Gemische davon sind im Handel erhältlich.

Die erfindungsgemäßen Dielektrika enthalten etwa 0,1 bis 30, vorzugsweise 2 bis 25% einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur. Wenn dabei eine Temperatur oberhalb des Klärpunkts des Hauptbestandteils gewählt wird, kann die Vollständigkeit des Lösungsvorgangs besonders leicht beobachtet werden.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann geläufig und in der Literatur ausführlich beschrieben. Beispielsweise können dichroitische Farbstoffe oder Substanzen zur Veränderung der dielektrischen Anisotropie, der Viskosität, der Leitfähigkeit und/oder der Orientierung der nematischen Phasen zugesetzt werden. Derartige Substanzen sind z. B. in den DE-OS 2 209 127, 2 240 864, 2 321 632, 2 338 281, 2 450 088, 2 637 430, 2 853 728 und 2 902 177 beschrieben.

Die folgenden Beispiele sollen die Erfindung erläutern. In den Beispielen bedeuten F. den Schmelzpunkt und K. den Klärpunkt einer flüssigkristallinen Substanz. Vor- und nachstehend bedeuten Prozentangaben Gewichtsprozent; alle Temperaturen sind in Grad Celsius angegeben.

## Beispiel 1

Man kocht 35,2 g trans,trans-4'-Propyl-bicyclohexyl-4-carbonsäure 1 Std. mit 24 g $SOCl_2$, dampft ein, löst das erhaltene rohe Säurechlorid in 150 ml Toluol, versetzt mit 7,9 g Pyridin und 6 g Propanol und kocht 2 Stunden. Das Gemisch wird gekühlt, mit Wasser gewaschen, die organische Phase wird über $Na_2SO_4$ getrocknet und eingedampft. Man erhält trans,trans-4'-Propyl-bicyclohexyl-4-carbonsäure-propylester, F. 24°, K. 54°.

## Beispiele 2 bis 43

Analog Beispiel 1 erhält man durch Veresterung der entsprechenden Carbonsäuren über die entsprechenden Säurechloride:

2. trans,trans-4'-Ethyl-bicyclohexyl-4-carbonsäure-ethylester.
3. trans,trans-4'-Ethyl-bicyclohexyl-4-carbonsäure-R-2-methylbutylester.
4. trans,trans-4'-Ethyl-bicyclohexyl-4-carbonsäure-S-2-methylbutylester.
5. trans,trans-4'-Ethyl-bicyclohexyl-4-carbonsäure-R-2-octylester.
6. trans,trans-4'-Ethyl-bicyclohexyl-4-carbonsäure-S-2-octylester.

7. trans,trans-4'-Propyl-bicyclohexyl-4-carbonsäure-ethylester.
8. trans,trans-4'-Propyl-bicyclohexyl-4-carbonsäure-butylester, F. 25°, K. 41°.
9. trans,trans-4'-Propyl-bicyclohexyl-4-carbonsäure-pentylester.
10. trans,trans-4'-Propyl-bicyclohexyl-4-carbonsäure-R-2-methylbutylester.
11. trans,trans-4'-Propyl-bicyclohexyl-4-carbonsäure-S-methylbutylester.
12. trans,trans-4'-Propyl-bicyclohexyl-4-carbonsäure-R-2-octylester.
13. trans,trans-4'-Propyl-bicyclohexyl-4-carbonsäure-S-2-octylester.
14. trans,trans-4'-Propyl-bicyclohexyl-4-carbonsäure-octylester.
15. trans,trans-4'-Propyl-bicyclohexyl-4-carbonsäure-trifluormethylester.
16. trans,trans-4'-Propyl-bicyclohexyl-4-carbonsäure-pentafluorethylester.
17. trans,trans-4'-Propyl-bicyclohexyl-4-carbonsäure-heptafluorpropylester.
18. trans,trans-4'-Propyl-bicyclohexyl-4-carbonsäure-nonafluorbutylester.
19. trans,trans-4'-Propyl-bicyclohexyl-4-carbonsäure-(trans-4-(trans-4-propylcyclohexyl)-cyclohexylester).
20. trans,trans-4'-Propyl-bicyclohexyl-4-carbonsäure-(trans-4-p-methoxyphenyl-cyclohexylester).
21. trans,trans-4'-Propyl-bicyclohexyl-4-carbonsäure-(p-trans-4-pentylcyclohexyl-phenylester).
22. trans,trans-4'-Propyl-bicyclohexyl-4-carbonsäure-(4'-propyloxy-4-biphenylyl-ester).
23. trans,trans-4'-Butyl-bicyclohexyl-4-carbonsäure-ethylester.
24. trans,trans-4'-Butyl-bicyclohexyl-4-carbonsäure-propylester.
25. trans,trans-4'-Butyl-bicyclohexyl-4-carbonsäure-pentylester.
26. trans,trans-4'-Butyl-bicyclohexyl-4-carbonsäure-R-2-methylbutylester, K. 47°.
27. trans,trans-4'-Butyl-bicyclohexyl-4-carbonsäure-S-2-methylbutylester.
28. trans,trans-4'-Butyl-bicyclohexyl-4-carbonsäure-R-2-octylester, Sdp. 210°/0,2 bar.
29. trans,trans-4'-Butyl-bicyclohexyl-4-carbonsäure-S-2-octylester.
30. trans,trans-4'-Pentyl-bicyclohexyl-4-carbonsäure-ethylester.
31. trans,trans-4'-Pentyl-bicyclohexyl-4-carbonsäure-propylester, F. 37°, K. 79°.
32. trans,trans-4'-Pentyl-bicyclohexyl-4-carbonsäure-pentylester.
33. trans,trans-4'-Pentyl-bicyclohexyl-4-carbonsäure-R-2-methylbutylester.
34. trans,trans-4'-Pentyl-bicyclohexyl-4-carbonsäure-S-2-methylbutylester.
35. trans,trans-4'-Pentyl-bicyclohexyl-4-carbonsäure-R-2-octylester.
36. trans,trans-4'-Pentyl-bicyclohexyl-4-carbonsäure-S-2-octylester.
37. trans,trans-4'-Heptyl-bicyclohexyl-4-carbonsäure-ethylester.
38. trans,trans-4'-Heptyl-bicyclohexyl-4-carbonsäure-propylester.
39. trans,trans-4'-Heptyl-bicyclohexyl-4-carbonsäure-pentylester.
40. trans,trans-4'-Heptyl-bicyclohexyl-4-carbonsäure-R-2-methylbutylester.
41. trans,trans-4'-Heptyl-bicyclohexyl-4-carbonsäure-S-2-methylbutylester.
42. trans,trans-4'-Heptyl-bicyclohexyl-4-carbonsäure-R-2-octylester.
43. trans,trans-4'-Heptyl-bicyclohexyl-4-carbonsäure-S-2-octylester.

### Beispiel 44

Analog Beispiel 1 erhält man aus Butyrylchlorid und trans,trans-4'-Butyl-bicyclohexyl-4-ol (herstellbar durch Hydrierung von p-trans-4-Butylcyclohexyl-phenol und anschließende Isomerentrennung das trans,trans-4'-Butyl-bicyclohexyl-4-butyrat, F. 24°, K. 86°.

### Beispiele 45 bis 64

Analog Beispiel 44 sind erhältlich:

45. trans,trans-4'-Methyl-bicyclohexyl-4-acetat.
46. trans,trans-4'-Ethyl-bicyclohexyl-4-acetat.
47. trans,trans-4'-Ethyl-bicyclohexyl-4-propionat.
48. trans,trans-4'-Ethyl-bicyclohexyl-4-butyrat.
49. trans,trans-4'-Ethyl-bicyclohexyl-4-capronat.
50. trans,trans-4'-Propyl-bicyclohexyl-4-acetat.
51. trans,trans-4'-Propyl-bicyclohexyl-4-propionat.
52. trans,trans-4'-Propyl-bicyclohexyl-4-butyrat.
53. trans,trans-4'-Propyl-bicyclohexyl-4-capronat.
54. trans,trans-4'-Butyl-bicyclohexyl-4-acetat.
55. trans,trans-4'-Butyl-bicyclohexyl-4-propionat.
56. trans,trans-4'-Butyl-bicyclohexyl-4-capronat.

57. trans,trans-4'-Pentyl-bicyclohexyl-4-acetat.
58. trans,trans-4'-Pentyl-bicyclohexyl-4-propionat.
59. trans,trans-4'-Pentyl-bicyclohexyl-4-butyrat, F. 33°, K. 93°.
60. trans,trans-4'-Pentyl-bicyclohexyl-4-capronat.
61. trans,trans-4'-Heptyl-bicyclohexyl-4-acetat.
62. trans,trans-4'-Heptyl-bicyclohexyl-4-propionat.
63. trans,trans-4'-Heptyl-bicyclohexyl-4-butyrat.
64. trans,trans-4'-Heptyl-bicyclohexyl-4-capronat.

Beispiel 65

Ein Gemisch von 3,56 g p-[trans-4-(trans-4-Propylcyclohexyl)-cyclohexylcarbonyloxy]-benzaldehyd (erhältlich durch Acylierung von p-Hydroxybenzaldehyd), 1,32 g 2-Butylpropan-1,3-diol, 0,01 g p-Toluolsulfonsäure und 20 ml Toluol wird am Wasserabscheider 4 Std. gekocht, abgekühlt, mit Wasser gewaschen und eingedampft. Man erhält 5-Butyl-2-p-[trans-4-(trans-4-propyl-cyclohexyl)-cyclohexylcarbonyloxy]-phenyl-1,3-dioxan.

Die folgenden Beispiele sind solche für erfindungsgemäße Dielektrika mit einem Gehalt an mindestens einer Verbindung der Formel I:

Beispiel A

Ein Gemisch aus

20% trans,trans-4'-Pentyl-bicyclohexyl-4-carbonsäure-propylester
25% p-(trans-4-Propyl-cyclohexyl)-benzonitril
20% trans,trans-4'-Butyl-bicyclohexyl-4-carbonitril
18% p-(trans-4-Propyl-cyclohexyl)-ethoxybenzol und
17% trans-4-Pentylcyclohexancarbonsäure-(p-trans-4-propylcyclohexyl-phenyl-ester)

zeigt F. 0°, K. 58°.

Beispiel B

Ein Gemisch aus

2% trans,trans-4'-Butylbicyclohexyl-4-carbonsäure-S-2-octylester
22% p-trans-4-Propylcyclohexyl-benzonitril
36% p-trans-4-Pentylcyclohexyl-benzonitril
25% p-trans-4-Heptylcyclohexyl-benzonitril und
15% 4'-(trans-4-Pentylcyclohexyl)-biphenyl-4-carbonitril

zeigt F. −5°, K. 69°.

**Patentansprüche**

1. Bicyclohexylderivate der Formel I

$$R^1-Cy-Cy-X-Y \qquad (I)$$

worin

X −CO−O− oder −O−CO− und
Y Alkyl oder Perfluoralkyl mit jeweils 1−8 C-Atomen, $-Cy-Cy-R^2$, $-Cy-Ph-R^2$, $-Ph-Cy-R^2$, $-Ph-Ph-R^2$ oder $-Ph-Dio-R^2$,
Cy 1,4-Cyclohexylen,
Ph 1,4-Phenylen,
Dio 1,3-Dioxan-2,5-diyl und
$R^1$ und $R^2$ jeweils Alkyl oder Alkoxy mit jeweils 1−8 C-Atomen

bedeuten.

2. Verfahren zur Herstellung von Bicyclohexylderivaten der Formel I nach Anspruch 1, dadurch gekennzeichnet, daß man eine Carbonsäure der Formel II

Q—COOH (II)

worin

Q (a) $R^1$—Cy—Cy— oder
(b) Y bedeutet und
$R^1$, Cy und Y die angegebene Bedeutung haben

oder eines ihrer reaktionsfähigen Derivate mit einer Hydroxyverbindung der Formel III

Z—OH (III)

worin

Z (a) Y oder
(b) $R^1$—Cy—Cy— bedeutet und
$R^1$, Cy und Y die angegebene Bedeutung haben

oder einem ihrer reaktionsfähigen Derivate umsetzt
oder daß man zur Herstellung von Verbindungen der Formel I (Y = —Ph—Dio—$R^2$) einen Aldehyd der Formel IV

$R^1$—Cy—Cy—X—Ph—CHO (IV)

worin $R^1$, X, Ph und Cy die angegebene Bedeutung haben
mit einem Diol der Formel V

$(HOCH_2)_2$—CH—$R^2$ (V)

worin $R^2$ die angegebene Bedeutung hat
umsetzt.

3. Verwendung der Verbindungen der Formel I nach Anspruch 1 als Komponenten flüssigkristalliner Dielektrika für elektrooptische Anzeigeelemente.

4. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel I nach Anspruch 1 ist.

5. Elektrooptische Anzeigeelemente, dadurch gekennzeichnet, daß es ein Dielektrikum nach Anspruch 4 enthält.

**Claims**

1. Bicyclohexyl derivatives of the formula I

$R^1$—Cy—Cy—X—Y (I)

wherein

X is —CO—O— or —O—CO—,
Y is alkyl or perfluoroalkyl with in each case 1—8 C atoms, —Cy—Cy—$R^2$, —Cy—Ph—$R^2$, —Ph—Cy—$R^2$, —Ph—Ph—$R^2$ or —Ph—Dio—$R^2$.
Cy is 1,4-cyclohexylene,
Ph is 1,4-phenylene,
Dio is 1,3-dioxane-2,5-diyl and
$R^1$ and $R^2$ are each alkyl or alkoxy with in each case 1—8 C atoms.

2. Process for the preparation of bicyclohexyl derivatives of the formula I according to Claim 1, characterised in that a carboxylic acid of the formula II

Q—COOH (II)

wherein

Q is
(a) $R^1$—Cy—Cy— or

(b) Y, and
$R^1$, Cy and Y have the meanings given,

or one of its reactive derivatives, is reacted with a hydroxy compound of the formula III

Z—OH (III)

wherein

Z is
(a) Y or
(b) $R^1-Cy-Cy-$, and
$R^1$, Cy and Y have the meanings given,

or one of its reactive derivatives, or in that, to prepare compounds of the formula I (Y = $-Ph-Dio-R^2$), an aldehyde of the formula IV

$R^1-Cy-Cy-X-Ph-CHO$ (IV)

wherein $R^1$, X, Ph and Cy have the meanings given, is reacted with a diol of the formula V

$(HOCH_2)_2-CH-R^2$ (V)

wherein $R^2$ has the meaning given.

3. Use of the compounds of the formula I according to Claim 1 as components of liquid crystal dielectrics for electrooptical display elements.

4. Liquid crystal dielectric for electrooptical display elements having at least two liquid crystal components, characterised in that at least one component is a compound of the formula I according to Claim 1.

5. Electrooptical display element, characterised in that it contains a dielectric according to Claim 4.

**Revendications**

1. Dérivés bicyclohexyliques de formule I

$R^1-Cy-Cy-X-Y$ (I)

dans laquelle

X représente —CO—O— ou —O—CO— et
Y représente un groupe alkyle ou perfluoralkyle contenant chacun 1 à 8 atomes de carbone, $-Cy-Cy-R^2$, $-Cy-Ph-R^2$, $-Ph-Cy-R^2$, $-Ph-Ph-R^2$ ou $-Ph-Dio-R^2$,
Cy représente un groupe 1,4-cyclohexylène,
Ph représente un groupe 1,4-phénylène,
Dio représente un groupe 1,3-dioxanne-2,5-diyle, et
$R^1$ et $R^2$ représentent chacun un groupe alkyle ou alcoxy contenant chacun 1 à 8 atomes de carbone.

2. Procédé de préparation des dérivés bicyclohexyliques de formule I selon la rev. 1, caractérisé en ce que l'on fait réagir un acide carboxylique de formule II

Q—COOH (II)

dans laquelle

Q représente
(a) $R^1-Cy-Cy-$ ou
(b) Y, et
$R^1$, Cy et Y ont les significations indiquées ci-dessus,

ou un dérivé réactif d'un tel acide, avec un composé hydroxylé de formule III

Z—OH (III)

dans laquelle

Z représente
(a) Y ou
(b) $R^1-Cy-Cy-$ et
$R^1$, Cy et Y ont les significations indiquées ci-dessus,

ou un dérivé réactif d'un tel composé,
ou bien, pour la préparation des composés de formule I ($Y = -Ph-Dio-R^2$) on fait réagir un aldéhyde de formule IV

$$R^1-Cy-Cy-X-Ph-CHO \qquad (IV)$$

dans laquelle $R^1$, X, Ph et Cy ont les significations indiquées ci-dessus,
avec un diol de formule V

$$(HOCH_2)_2-CH-R^2 \qquad (V)$$

dans laquelle $R^2$ a la signification indiquée ci-dessus.

3. Utilisation des composés de formule I selon 1a rev. 1 en tant que composants de diélectriques à cristaux liquides pour des éléments d'affichage électrooptiques.

4. Diélectrique à cristaux liquides pour éléments d'affichage électro-optiques contenant au moins deux composants à cristaux liquides, caractérisé en ce que l'un au moins des composants est un composé de formule I selon 1a rev. 1.

5. Elément d'affichage électro-optique, caractérisé en ce qu'il contient un diélectrique selon 1a rev.4.